(19) Europäisches Patentamt
European Patent Office
Office européen des brevets

(11) Veröffentlichungsnummer: **0 262 639 B1**

(12) **EUROPÄISCHE PATENTSCHRIFT**

(45) Veröffentlichungstag der Patentschrift: **12.06.91**

(51) Int. Cl.$^5$: **C07C 409/16**, C07C 407/00

(21) Anmeldenummer: **87114145.3**

(22) Anmeldetag: **28.09.87**

# (54) Verfahren zur Herstellung von t-Alkyl-t-aralkylperoxiden.

(30) Priorität: **30.09.86 DE 3633308**

(43) Veröffentlichungstag der Anmeldung:
**06.04.88 Patentblatt 88/14**

(45) Bekanntmachung des Hinweises auf die Patenterteilung:
**12.06.91 Patentblatt 91/24**

(84) Benannte Vertragsstaaten:
**BE DE ES FR GB IT NL**

(56) Entgegenhaltungen:
**GB-A- 954 361**

(73) Patentinhaber: **Peroxid-Chemie GmbH**
**Dr.-Gustav-Adolph-Strasse 3**
**W-8023 Höllriegelskreuth bei München(DE)**

(72) Erfinder: **Hägel, Eberhard, Dr.**
**Eichendorffweg 26a**
**W-8021 Icking(DE)**

(74) Vertreter: **Huber, Bernhard, Dipl.-Chem. et al**
**Patentanwälte H. Weickmann, Dr. K. Fincke**
**F.A. Weickmann, B. Huber Dr. H. Liska, Dr. J.**
**Prechtel Möhlstrasse 22 Postfach 860 820**
**W-8000 München 86(DE)**

EP 0 262 639 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von t-Alkyl-t-aralkylperoxiden der allgemeinen Formel I

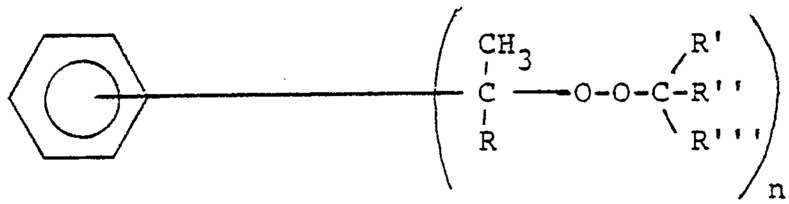

wobei R einen Alkylrest mit 1 bis 4 C-Atomen und R', R'' und R''' gleiche oder verschiedene Alkylreste mit 1 bis 5 C-Atomen bedeuten und n eine Zahl von 1 bis 3 ist, indem man ein Olefin der allgemeinen Formel II

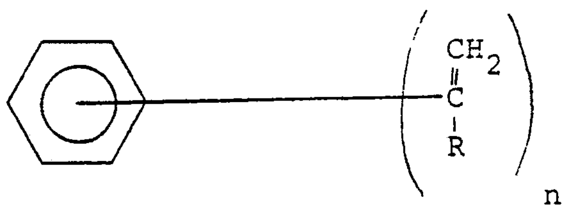

mit einem t-Alkylhydroperoxid der allgemeinen Formel III:

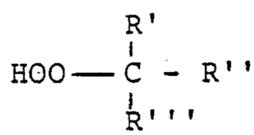

worin R, R', R'' und R''' sowie n die oben angegebene Bedeutung haben, umsetzt.

t-Alkyl-t-aralkylperoxide können bei relativ hohen Temperaturen relativ stabile Radikale bilden und sind daher besonders geeignet als Initiatoren für die Vernetzung von Kunststoffen. Sie haben weiterhin den Vorteil, daß sie keine Zersetzungsprodukte bilden, die störende Eigenschaften haben. Verfahren zu ihrer Herstellung sind daher schon seit längerem bekannt. Weit verbreitet sind insbesondere Verfahren, bei denen ein tertiär-Alkylhydroperoxid mit dem entsprechenden Carbinol kondensiert wird. Nachteil dieses Verfahrens ist, daß die Carbinole erst aus den entsprechenden Hydroperoxiden durch Reduktion hergestellt werden müssen und daß es sich im Falle der Diole und Triole um feste Verbindungen handelt, die schwierig zu dosieren sind.

Bekannt ist weiterhin z.B. aus DE-OS 20 16 108, t-Alkyl-t-aralkylperoxide herzustellen durch Reaktion von Olefinen der allgemeinen Formel II mit Hydroperoxiden unter Säurekatalyse. Wesentlich für dieses Verfahren ist es, daß das Milieu völlig wasserfrei ist. Es muß daher sowohl ein wasserfreies Hydroperoxid als auch die Säure in wasserfreiem Zustand eingesetzt werden, wobei bei Verwendung von Salzsäure dies den Einsatz von gasförmigem Chlorwasserstoff bedeutet. Praktisch wasserfreies Hydroperoxid ist aufwendig herzustellen und gefährlich. Darüberhinaus erfordert dieses bekannte Verfahren lange Reaktionszeiten und führt trotzdem nur zu schlechten Ausbeuten mit einer geringen Reinheit des erhaltenen Produktes.

Es war daher Aufgabe der vorliegenden Erfindung, ein Verfahren zur Herstellung von t-Alkyl-t-aralkylperoxiden zu schaffen, bei dem der Einsatz von gasförmigem Chlorwasserstoff und wasserfreiem Hydroperoxid nicht notwendig ist, das in kurzer Reaktionszeit mit hoher Ausbeute durchgeführt werden kann.

Diese Aufgabe wird gelöst durch ein Verfahren zur Herstellung von t-Alkyl-t-aralkylperoxiden der allgemeinen Formel I

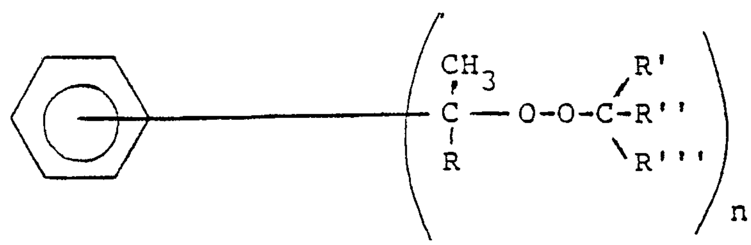

wobei R einen Alkylrest mit 1 bis 4 C-Atomen und R', R'' und R''' gleiche oder verschiedene Alkylreste mit 1 bis 5 C-Atomen bedeuten und n eine Zahl von 1 bis 3 ist, indem man ein Olefin der allgemeinen Formel II

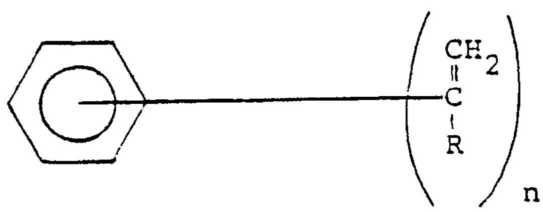

mit einem t-Alkylhydroperoxid der allgemeinen Formel III

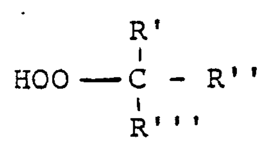

worin R, R', R'' und R''' sowie n die oben angegebene Bedeutung haben, umsetzt, das dadurch gekennzeichnet ist, daß man die Umsetzung in Gegenwart von wäßriger Salzsäure durchführt.

Überraschenderweise ist es erfindungsgemäß möglich, in kurzer Zeit mit hoher Ausbeute und hoher Reinheit t-Alkyl-t-aralkylperoxide herzustellen. Für das erfindungsgemäße Verfahren können verdünnte Salzsäure sowie wasserhaltiges Hydroperoxid verwendet werden.

Bevorzugt wird als Olefin die Verbindung der Formel II, in der n gleich 1 ist, also alpha-Methylstyrol verwendet.

Bevorzugt sind die Reste R, R', R'' und R''' Alkylreste mit 1 oder 2 C-Atomen.

Bei der Durchführung des erfindungsgemäßen Verfahrens sollte das t-Alkylhydroperoxid im Überschuß gegenüber dem Olefin vorliegen. Bevorzugt werden 1,5 bis 2 Mol t-Alkylhydroperoxid pro Mol olefinischer Doppelbindung eingesetzt.

Die Additionsreaktion wird in Gegenwart von wäßriger Salzsäure durchgeführt. Die Konzentration der wäßrigen Salzsäure ist nicht kritisch. Es kann jede handelsübliche verdünnte Salzsäure verwendet werden. Bevorzugt liegt die Konzentration der Salzsäure in einem Bereich von 20 bis 40 %, besonders bevorzugt 25 bis 37 %.

Die Salzsäure wird, bezogen auf eingesetzte Olefingruppen, in einer Menge von 0,5 bis 2 Mol verwendet.

Die Umsetzung kann bei Umgebungstemperatur durchgeführt werden. Bevorzugt erfolgt die Zugabe der Salzsäure bei einer Temperatur zwischen 15 und 35 °C. Während der Reaktion steigt die Temperatur an, da die Reaktion exotherm ist. Die Umsetzung erfolgt bei normalem Druck.

Die Abtrennung des Reaktionsproduktes erfolgt in an sich bekannter Weise. Wenn das Peroxid kristallin anfällt, kann es durch Abfiltrieren oder Zentrifugieren von der wäßrigen Phase getrennt werden. Fällt es flüssig an, so kann die organische von der wässrigen Phase durch Dekantieren abgetrennt werden. Nach der Abtrennung ist es zweckmäßig, das erhaltene Produkt alkalisch zu waschen.

Das erfindungsgemäße Verfahren liefert die gewünschten t-Alkyl-t-aralkylperoxide mit einer Reinheit von mehr als 95 %. Die Ausbeuten liegen dabei über 90 %. Die Verwendung von handelsüblicher verdünnter Salzsäure und von handelsüblichem wasserhaltigem Butylhydroperoxid sind weitere Vorteile des erfindungsgemäßen Verfahrens. Es wird somit ein Verfahren zur Verfügung gestellt, das in kurzer Zeit und auf einfache Weise das gewünschte Produkt in hoher Ausbeute und in hoher Reinheit liefert.

Die Erfindung wird noch durch Beispiele erläutert.

**Beispiel 1**

Zu einer Mischung von 238 g alpha-Methylstyrol (2 Mol) und 394 g 80%igem t-Butylhydroperoxid (3,5 Mol) dosiert man unter Rühren innerhalb von 15 min 235 g 31%ige Salzsäure (2 Mol), wobei man die Temperatur der Mischung von 20° C auf 35° C steigen läßt. Anschließend rührt man noch 2 Stunden bei 40° C nach, gibt dann 250 ml Wasser zu, läßt 15 min stehen und trennt die wäßrige Phase ab. Die organische Phase wird mit 250 ml 15%iger Natronlauge und zweimal mit je 500 ml Wasser gewaschen. Nach Zusatz von 20 g Wasser wird bei 50° C mit Luft ausgeblasen (30 min). Man erhält 395 g einer schwach gelblichen Flüssigkeit mit einem Gehalt von 96 % t-Butylcumylperoxid und weniger als 0,4 % alpha-Methylstyrol. Das entspricht einer Ausbeute von 91 % bezogen auf eingesetztes alpha-Methylstyrol.

**Beispiel 2**

In 900 g 80% t-Butylhydroperoxid (8 Mol) löst man 317 g 1.4-Diisopropenylbenzol (2 Mol), gibt 1 ml eines Netzmittels (Dodecylbenzolsulfonsäure) zu und fügt unter Rühren bei 35° C 470 g 31 % Salzsäure (4 Mol) zu, wobei die Temperatur der Mischung bis 45° C steigt. Nach etwa 10 Minuten beginnt das Peroxid auszukristallisieren, man rührt noch 20 Minuten bei 40° C nach und trennt dann die Mutterlauge weitgehend vom aufschwimmenden Peroxid ab. Man gibt 1 1 12 % Natronlauge zu, rührt 10 Minuten bei 30 bis 40° C, kühlt auf 20° C ab und filtriert. Der Feststoff wird auf der Nutsche mit Wasser neutral gewaschen und bei Raumtemperatur getrocknet. Man erhält 590 g eines fast weißen Pulvers mit einem Gehalt von 96 % an 1.4-Bis-(t-butylperoxy-isopropyl)-benzol (84 % der theoretischen Ausbeute).

**Vergleichsbeispiel 3**

Gemäß Beispiel 3 der GB-PS 954 361 wurden 0,1 Mol trockenes Chlorwasserstoffgas in 1,15 Mol alpha-Methylstyrol umgesetzt mit 1 Mol 98 %igem t-Butylhydroperoxid. Die Umsetzung dauerte 5 Stunden und wurde bei 50° C durchgeführt. Nach Waschen und Trocknen wurden 177,3 g Rohprodukt erhalten, die gemäß GC-Analyse einen Gehalt von 70,7 % t-Butylcumylperoxid und 25,8 % nicht umgesetztes alpha-Methylstyrol neben kleinen Anteilen anderer Verunreinigungen hatten. Durch Ausblasen mit Luft bei 50° C nach Zusatz von 5 Gew.-% Wasser erhielt man 145 g einer Flüssigkeit mit einem Gehalt von 83,9 % t-Butylcumylperoxid und 13 % alpha-Methylstyrol. Das entspricht einer Ausbeute an t-Butylcumylperoxid von 58 % bezogen auf eingesetztes t-Butylhydroperoxid bzw. von 50 % bezogen auf eingesetztes alpha-Methylstyrol.

Es wird somit bei den bekannten Verfahren trotz Einsatz von 98%igem t-Butylhydroperoxid bei langer Reaktionszeit eine schlechte Ausbeute und eine geringere Reinheit des Produktes erhalten.

## Ansprüche

1. Verfahren zur Herstellung von t-Alkyl-t-aralkylperoxiden der allgemeinen Formel I

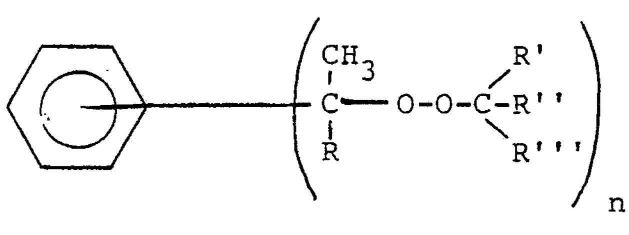

wobei R einen Alkylrest mit 1 bis 4 C-Atomen und R', R" und R'" gleiche oder verschiedene Alkylreste mit 1 bis 5 C-Atomen bedeuten und n eine Zahl zwischen 1 und 3 ist, indem man ein Olefin der allgemeinen Formel II

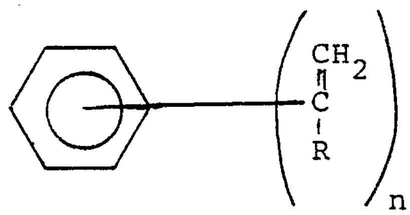

mit einem t-Alkylhydroperoxid der allgemeinen Formel III

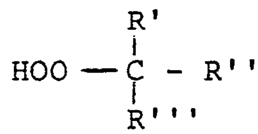

worin R, R', R" und R'" sowie n die oben angegebene Bedeutung haben, umsetzt,
**dadurch gekennzeichnet,**
daß man die Umsetzung in Gegenwart von wäßriger Salzsäure durchführt.

**2.** Verfahren nach Anspruch 1,
**dadurch gekennzeichnet,**
daß die Salzsäure in einer Menge von 0,5 bis 2 Mol pro Mol olefinischer Doppelbindung eingesetzt wird.

**3.** Verfahren nach Anspruch 1 oder 2,
**dadurch gekennzeichnet,**
daß die Salzsäure mit einer Konzentration von 20 bis 40 % eingesetzt wird.

**4.** Verfahren nach Anspruch 3,
**dadurch gekennzeichnet,**
daß die Salzsäure mit einer Konzentration von 25 bis 37 % eingesetzt wird.

## Claims

**1.** Process for the preparation of t.-alkyl-t.-aralkyl peroxides of the general formula I

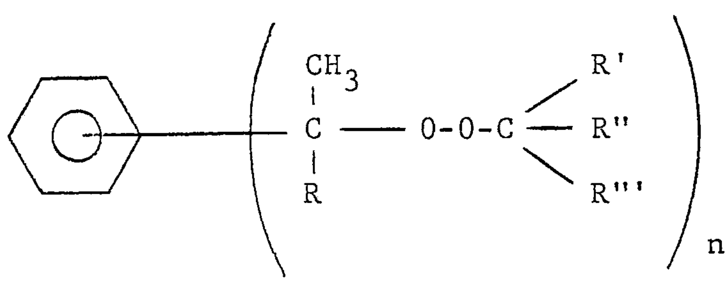

whereby R signifies an alkyl radical with 1 to 4 C-atoms and R', R" and R'" signify the same or different alkyl radicals with l to 5 C-atoms and n is a number between 1 and 3, in that one reacts an olefin of the general formula II

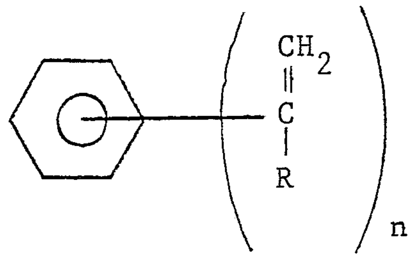

with a t.-alkyl hydroperoxide of the general formula III

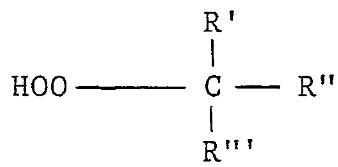

wherein R, R', R''' and R''', as well as n, have the above-given meaning, characterised in that one carries out the reaction in the presence of aqueous hydrochloric acid.

2. Process according to claim 1, characterised in that the hydrochloric acid is used in an amount of 0.5 to 2 mole per mole of olefinic double bond.

3. Process according to claim 1 or 2, characterised in that the hydrochloric acid is used with a concentration of 20 to 40%.

4. Process according to claim 3, characterised in that the hydrochloric acid is used with a concentration of 25 to 37%.

**Revendications**

1. Procédé de préparation de peroxydes de t-alkyle-t-aralkyle répondant à la formule générale I

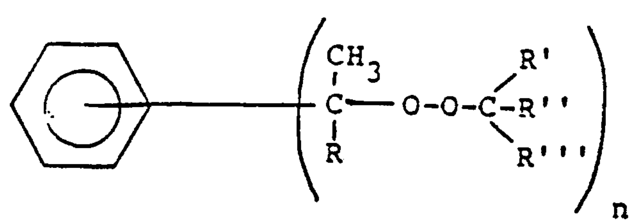

dans laquelle R désigne un radical alkyle en $C_1$ et $C_4$ et R', R'' et R''' designent des radicaux alkyle en C1 à C5 identiques ou différents et n est un nombre entre 1 et 3, en faisant réagir une oléfine répondant à la formule générale II

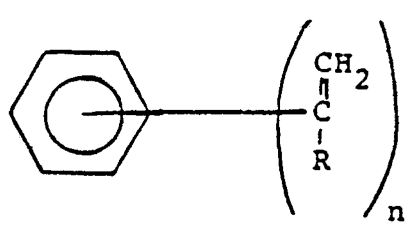

avec un hydroperoxyde de t-alkyle répondant à la formule générale III

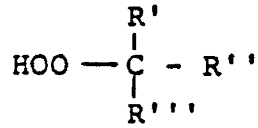

dans laquelle R, R', R'' et R''' ainsi que n ont la signification indiquée ci-dessus, caractérisé en ce qu'on effectue la réaction en présence d'acide chlorhydrique aqueux.

2. Procédé selon la revendication 1, caractérisé en ce que l'acide chlorhydrique est utilisé dans une quantité de 0,5 à 2 moles par mole de doubles liaisons oléfiniques.

3. Procédé selon les revendications 1 ou 2, caractérisé en ce que l'acide chlorhydrique est utilisé à une concentration de 20 à 40 %.

4. Procédé selon la revendication 3, caractérisé en ce que l'acide chlorhydrique est utilisé à une concentration de 25 à 37 %.